# EUROPÄISCHE PATENTSCHRIFT

(11) **EP 3 077 800 B1**
(45) Veröffentlichungstag und Bekanntmachung des Hinweises auf die Patenterteilung: **19.10.2022**
(21) Anmeldenummer: 14805939.7
(22) Anmeldetag: 03.12.2014
(51) Int. Cl.: G01N 21/64, C12Q 1/04, C12Q 1/18, G01N 33/569

(54) **VERFAHREN UND VORRICHTUNG ZUR ERFASSUNG RESISTENTER KEIME**
METHOD AND DEVICE FOR DETECTING RESISTANT MICROBES
PROCÉDÉ ET DISPOSITIF DE DÉTECTION DE GERMES RÉSISTANTS

(30) Priorität: 05.12.2013 DE 102013225037
(43) Veröffentlichungstag der Anmeldung: 12.10.2016
(73) Patentinhaber: Fraunhofer-Gesellschaft zur Förderung der angewandten Forschung e.V., 80686 München (DE); Asklepios Kliniken Verwaltungsgesellschaft mbH, 61462 Königstein (DE)
(72) Erfinder: SCHMIDT, Jennifer, 82445 Schwaigen-Grafenaschau (DE); EISELE, Ignaz, 82057 Icking (DE); TRUPP, Sabine, 07318 Saalfeld (DE); HABERGER, Karl, 82152 Martinsried (DE); SITTEL, Wolfgang, 61350 Bad Homburg (DE)
(74) Vertreter: Zimmermann, Tankred Klaus
(86) Internationale Anmeldenummer: PCT/EP2014/076362
(87) Internationale Veröffentlichungsnummer: WO 2015/082523

(56) Entgegenhaltungen:
- WO-A1-99/18232
- WO-A1-2006/092629
- WO-A1-2010/129779
- WO-A2-2006/077586

## Beschreibung

Die Erfindung bezieht sich auf ein Verfahren zur Erkennung resistenter Keime in einem Probenmaterial und eine Vorrichtung zur Durchführung des Verfahrens.

Eine Vielzahl unterschiedlicher Bakterien gehören zur physiologischen Flora der Haut und Schleimhäute und sind damit Teil des Mikrobioms, also der Gesamtheit aller den Menschen besiedelnden Mikroorganismen. Durch die Besiedelung übernehmen die Bakterien wichtige Aufgaben, wie z.B. den Schutz der Haut und des gesamten Organismus vor dem Eindringen pathogener, also gesundheitsgefährdender Keime, da diese um ihren Lebensraum, den Menschen, konkurrieren. Gerät dieses System aus dem Gleichgewicht, beispielsweise durch eine Schwäche des Immunsystems, so kann das zur Entstehung von Infektionen führen. In der Regel lassen sich bakterielle Infektionen schnell und einfach durch die Gabe von Antibiotika behandeln.

Im Laufe der Jahre haben sich jedoch Resistenzen der Erreger gegen die eingesetzten Wirkstoffe entwickelt, wodurch die Therapie massiv erschwert wird. Keime und Erreger, die resistent gegen die eingesetzten Wirkstoffe sind, werden als multiresistente Erreger (MRE) bezeichnet. Das stellt besonders in Kliniken sowie in Alten- und Pflegeheimen ein großes Problem dar, denn hier befinden sich alte und kranke Menschen, deren Immunsystem geschwächt ist und die infolgedessen besonders anfällig für Infektionen sind. Auch Säuglinge, bei denen das Abwehrsystem noch nicht vollständig ausgebildet ist und immunsupprimierte Patienten, etwa auf einer Intensivstation, deren Immunreaktion durch die Einnahme von Medikamenten unterdrückt ist, beispielsweise infolge von Transplantationen, um Abstoßungsreaktionen zu minimieren, sind hier besonders gefährdet, sich mit multiresistenten Erregern zu infizieren.

Methicillin-resistente Staphylococcus aureus (MRSA) Stämme verursachen weltweit die meisten im Krankenhaus oder Alten- und Pflegeheim erworbenen Infektionen der Haut sowie Weichteilinfektionen. Häufig zeigen diese einen septischen Verlauf, was als besonders kritisch zu bewerten ist. Da MRSA nicht nur gegen das Penicillin Methicillin, sondern auch gegen die meisten anderen routinemäßig eingesetzten Antibiotika resistent ist, erfolgt hier eine Therapie mit sogenannten Reserveantibiotika. Dennoch ist der Heilungsverlauf oft sehr zeitintensiv und schwierig - wenn überhaupt erfolgreich. Neben dem Methicillin-resistenten Staphylococcus aureus gibt es auch Stämme, die gegen andere Antibiotika wie z.B. Vancomycin Resistenzen aufweisen. Auch andere Bakteriengattungen wie z.B. Enterokokken, Pneumokokken, Pseudomonas aeruginosa, Campylobacter, EHEC, u.a. zeigen mehrfache Resistenzen auf und sind daher ebenfalls als Problemkeim zu betrachten.

Eine Übertragung von MRE erfolgt entweder durch direkten Kontakt eines MRE-Trägers, bspw. bei Benutzung derselben Nasszelle eines MRE-Trägers, oder durch behandelnde Ärzte und Pflegepersonal, sofern die Hygienemaßnahmen, besonders die konsequente Händehygiene, nicht befolgt werden.

Zur Minimierung der Gefahr einer Weiterverbreitung der MRE sind besondere Hygienemaßnahmen einzuhalten. Behandelnde Ärzte und Pflegepersonal tragen Kittel, Mundschutz und Handschuhe und befolgen konsequent die hygienische Händedesinfektion (Barrierepflege). Die Unterbringung des Patienten sollte in einem Einzelzimmer erfolgen.

Zur Ergreifung und Umsetzung dieser Maßnahmen ist es jedoch notwendig, den MRE Trägerstatus eines Patienten schon bei der Aufnahme in eine Gesundheitseinrichtung zu kennen. Hierfür ist es ratsam, jede Neuaufnahme routinemäßig auf MRE zu screenen. Diese Untersuchung stellt eine wichtige präventive Maßnahme dar, nosokomiale Infektionen mit multiresistenten Erregern zu minimieren und im besten Fall zu verhindern.

In der klinischen Mikrobiologie werden bislang Untersuchungsmaterialien (z.B. Abstrichtupfer), die humanpathogene Keime tragen, auf geeigneten Universal- und Selektivmedien ausgestrichen bzw. in flüssige Nährbouillons eingeimpft. Nach einer bestimmten Inkubationszeit sind im Brutschrank Kolonien zu erkennen. Eine Zellteilung dauert bei den meisten Bakterien ca. 20 Minuten, so dass bis zu einem Heranwachsen einer hinreichend großen Anzahl von Bakterien typischerweise Tage, in einigen Fällen (z.B. Mycobacterium tuberkulosis) auch Wochen vergehen können. Die Erreger wachsen jedoch häufig in Mischkulturen, die anschließend eine Isolation einzelner Kolonien erfordern, um Reinkulturen zu erhalten. Diese werden in der Folge vermehrt, mikroskopiert und morphologisch beurteilt. Mithilfe verschiedener chromogener Selektivmedien, biochemischer Nachweisreaktionen und Spezialfärbungen werden die Keime identifiziert.

Häufig findet die Charakterisierung der Bakterien über den Nachweis einer keimspezifischen Enzymausstattung durch Farbreaktionen statt und erlaubt ihre Bestimmung und Zuordnung. Die Kultivierung und Charakterisierung der Bakterien dauert - je nach Wachstumsverhalten des jeweiligen Keims - Tage bis mehrere Wochen, wie es in [1, 2, 3] beschrieben ist.

Im Anschluss daran erfolgt die Erstellung eines Antibiogramms durch Auflegen von ausgewählten ggf. verschiedenen Antibiotikaplättchen auf festes Nährmedium, wie etwa aus Agar, auf dem die Bakterien zuvor ausgestrichen wurden. Wachstumshemmhöfe auf dem Agar zeigen an, ob und wie sensibel die Keime auf das entsprechende Antibiotikum der Plättchen reagieren. In der Regel ist hierfür eine 18 - 24-stündige Inkubation erforderlich, die jedoch bei bestimmten, meist anspruchsvollen Erregern aufgrund des langsamen Wachstums auch deutlich länger sein kann. Für einen Heilungsverlauf kann dies bedeuten, dass, bis eine Therapie ggf. angepasst werden kann, wertvolle Zeit vergeht. In anderen Worten diffundieren die Antibiotika während der 18 - 24 stündigen Inkubation in den Agar. Reagiert der Erreger sensibel auf ein Antibiotikum, entsteht ein Hemmhof um das aufgelegte Plättchen.

Alternativ zur zeitintensiven, konventionellen mikrobiologischen Anzucht der Erreger besteht auch die Möglichkeit, spezifische DNA-Sequenzen (Desoxyribonukleinsäure - DNS-, engl. deoxyribonucleic acid - DNA-) Sequenzen von multiresistenten Staphylococcus aureus Erregern mittels einer Polymerase-Kettenreaktion (Polymerase Chain Reaction - PCR) nachzuweisen. Eine nachzuweisende Zielsequenz kann beispielsweise das SCCmec-Gen sein. Der Nachweis dieser erregerspezifischen Zielsequenzen erfolgt zumeist über die klassische Polymerase-Kettenreaktion mit entsprechenden Primer-Sequenzen. Ist die DNA-sequenz, die zu den Primern komplementär ist, vorhanden, wird sie vervielfältigt. Durch Interaktion der DNA mit einem Fluoreszenz-Farbstoff, wie etwa SYBR Green I, wird die Reaktion sichtbar und messbar. Je mehr doppelsträngige DNA vorhanden ist, umso stärker ist das Fluoreszenzsignal.

Alternativ existieren auch Nachweismethoden, bei denen der Nachweis der Bakterien-DNA durch Hybridisierung mit fluoreszenz-gelabelten Target-Sequenzen erfolgt. Als Abstrichmaterial wird vom Robert-Koch Institut ein Nasen/Rachenabstrich des Patienten empfohlen. Die Sensitivität des MRSA-DNA-Nachweises liegt bei ca. 95 %, die Spezifität bei 97 - 99 %. Der molekularbiologische MRSA-Direktnachweis ist somit sehr keimspezifisch und die Durchführung dauert ca. 2 - 3 Stunden. Nachteilig ist jedoch, dass der molekularbiologische Nachweis durchschnittlich etwa 10-fach so teuer ist wie konventionelle Methoden und aus praktisch-organisatorischen Gründen erst nach Sammlung mehrerer Patientenproben mit gleicher Fragestellung zeitversetzt analysiert wird. Das kann erfahrungsgemäß am nächsten Tag geschehen, aber auch bis mehrere Tage nach der Probenabnahme dauern. Das hat zur Folge, dass bei Patientenaufnahme in die Gesundheitseirichtung noch gar kein Ergebnis bezüglich MRE-Trägerstatus vorliegt und somit zu diesem Zeitpunkt auch nicht entschieden werden kann, ob eine Isolation des Patienten notwendig ist oder nicht.

Als Therapieverlaufskontrolle ist der PCR-basierte Nachweis ungeeignet, da auch die DNA von nicht lebensfähigen Bakterien nachgewiesen wird. Eine Anzahl von lebenden Keimen bzw. eine Abnahme einer Anzahl lebender Keime, die eine Heilung andeuten kann, wird somit nicht detektiert. Weiterhin kann dieser Test nicht zur Vor-Ort-Erkennung eingesetzt werden und ist vergleichsweise teuer, wie es in [4, 5, 6] beschrieben ist.

Das Anlegen einer Kultur zur Erstellung eines Antibiogramms erfolgt in der Regel immer parallel zur Durchführung der molekularbiologischen Untersuchung, dauert - je nach Wachstumsverhalten des Erregers - mindestens einen Tag. Darüber hinaus kann diese Methode nur in Speziallabors mit hohem apparativem Aufwand von qualifiziertem Fachpersonal durchgeführt werden, für eine schnelle "bed-side" Analyse ist sie damit nicht geeignet.

Gegenüber PCR-Methoden existieren auch antikörper-basierte Nachweismethoden für Multiresistente Erreger, von denen einige im Nachfolgenden kurz erläutert werden.

Neben dem molekularbiologischen MRSA-Screening werden auch Bakteriophagenbasierte Technologien eingesetzt. Bakteriophagen sind Viren, die ganz spezifisch Bakterien befallen, Phagen-DNA in die Wirtszelle, den MRE, einbringen, deren Replikationsmaschinerie nutzen um ihr Virusgenom transkribieren zu lassen und nach Translation eigene Proteine herstellen zu lassen. Die Phagen vermehren sich innerhalb der Zelle und werden nach Zelllysis, dem Auflösen der Zellmembran nach dem Absterben der Zelle, freigesetzt. Die von den Phagen produzierten Proteine lassen sich mit gelabelten Antikörpern nachweisen und ermöglichen somit einen spezifischen Erregernachweis. Dieses Verfahren wird auch als Antikörper-basierte Nachweismethode bezeichnet. Bei dieser Methode nutzt man den Vorteil, dass die Vermehrung der Bakteriophagen deutlich schneller ist als die der Bakterienzellen selber, wie es in [7] beschrieben ist.

Eine weitere Strategie zum spezifischen MRSA-Nachweis ist der sogenannte Q-MAP-Test, wie er bspw. in [8] beschrieben ist. Hier werden Antikörper eingesetzt, die gegen bakterienspezifische Antigene gerichtet sind, die auf der Oberfläche der Erreger präsentiert werden, d. h. angeordnet sind. Die Antikörper sind an magnetische Nanopartikel gebunden und werden mit der entsprechenden Probe inkubiert. Kommt es zur Antigen-Antikörper-Reaktion, so werden Mikropräzipitate gebildet. Diese Immunkomplexe können messtechnisch von einem Q-MAP-System erfasst werden.

Grundsätzlich gelten für Antikörper-basierte Nachweisstrategien, dass sie - ähnlich zum molekularbiologischen Ansatz - sehr spezifisch sind. Jedoch ist die Gewinnung von Antikörpern aufwendig und bringt vergleichsweise hohe Herstellungskosten mit sich. Da es sich um Proteine handelt und diese temperaturempfindlich sind, erfordern Transport und Lagerung besondere Bedingungen. Auch die Lagerstabilität ist limitiert. Die Reproduzierbarkeit der Herstellung von Antikörpern in gleichbleibend guter Qualität innerhalb der einzelnen Chargen kann problematisch werden, was wiederum zu Engpässen in deren Verfügbarkeit führen kann.

Das Anlegen einer Kultur zur Erstellung eines Antibiogramms erfolgt in der Regel stets parallel zur Durchführung der molekularbiologischen Untersuchung und dauert, je nach Wachstumsverhalten des Erregers, mindestens einen Tag. Als universelles Eingangs-Screening ist der spezifische Erregernachweis somit nur bedingt geeignet. Da auch die DNA von nicht lebenden Erregern bei dem PCR-Nachweis erfasst wird, eignet sich diese Form des Nachweises auch nicht für eine Therapiekontrolle, die beispielsweise eine Konzentration von lebenden Erregern beschreibt.

In der Routine von Gesundheitseinrichtungen erfolgen derzeit Untersuchungen zur Erkennung von multiresistenten Erregern nur bei Verdachtspatienten. Die Nachweismethoden sind entweder sehr zeitaufwendig (mikrobiologisch-kultureller Nachweis) oder sehr kostenintensiv und spezifisch (molekularbiologischer oder Antikörper-basierter Nachweis).

In WO 2006/092629 A1 ist eine Probenahmevorrichtung zum Nachweis von Bakterien, beschrieben, die umfasst: ein Probenahmemedium zum Aufnehmen einer Bakterienprobe; und eine Vielzahl von Bakteriophagen. Die Bakteriophagen befinden sich auf oder im Probenahmemedium. Jeder Bakteriophage umfasst eine Nukleinsäure, die ein Protein codiert, das Licht mit einer Ausgangswellenlänge emittieren kann.

In WO 2006/077586 A2 ist ein kolorimetrischer und / oder fluoreszierender Detektor beschrieben, der einen Film aus Polydiacetyelenen und Lipiden umfasst, die auf einem Substrat abgeschieden sind. Der Film umfasst ferner mindestens ein keimtötendes Material. Mittels des Detektors können Antibiotikaresistenzen von mit dem Film in Kontakt gebrachten Bakterienstämmen bestimmt werden.

In WO 99/18232 A1 werden verschiedene Verfahren zur Erfassung resistenter Keime in einer Probe beschrieben. Bei dem Verfahren gemäß Beispiel 6 wird die Probe mit einem Probenträger kontaktiert, der ein Mittel zur Abtötung unterschiedlicher Keime, das mindestens ein Antibiotikum enthält, und ein fluorogenes Enzym-Substrat, das bei Reaktion mit einem von dem Keim produzierten Enzym eine fluoreszierende Einheit freisetzt, aufweist. Die resistenten Keime werden anhand von Fluoreszenzemissionen von dem Probenträger nachgewiesen.

In WO 2010/129779 A1 ist ein Verfahren und ein System zur Bestimmung des Antibiotikaresistenzstatus von Mikroorganismen beschrieben.

Wünschenswert wäre demnach ein Schnelltest zum MRE-Nachweis, der eine zügige Eingangsuntersuchung von Patienten auf multiresistente Erreger ermöglicht, um das Übertragungsrisiko der Erreger auf andere Menschen und Patienten deutlich zu minimieren.

Die Aufgabe der vorliegenden Erfindung besteht deshalb darin, ein Verfahren und eine das Verfahren implementierende Vorrichtung zu schaffen, das einen Nachweis von multiresistenten Keimen in einer Probe mit einem geringeren Zeitaufwand ermöglicht. Diese Aufgabe wird durch den Gegenstand der unabhängigen Patentansprüche gelöst. Der Kerngedanke der vorliegenden Erfindung besteht deshalb darin, erkannt zu haben, dass ein Verfahren, welches eine Probe mit einer Gesamtheit an Keimen auf eine Anwesenheit von gegen eine Mehrzahl von Antibiotika oder Antimykotika resistente Keimen unabhängig von deren Bakterien- oder Pilz-Stamm überprüft, eine zuverlässige und schnelle Aussage über eine Anwesenheit von multiresistenten Erregern in der Patientenprobe liefern kann, indem auf Kultivierungsschritte verzichtet wird. Gemäß Ausführungsbeispielen wird durch eine Implementierung des Verfahrens in einem Gerät mit einem hochempfindlichen Detektor bereits eine Anwesenheit einer geringen Anzahl an multiresistenten Erregern angezeigt, so dass eine weitere Beschleunigung der Auswertung ermöglicht ist.

Die Erfindung betrifft ein Verfahren zur Erfassung resistenter Keime in einer Probe durch Nachweis lebender resistenter Keime durch die Messung einer enzymatischen Aktivität der von den lebenden Keimen gebildeten Proteine DNase oder Hyaluronidase, bei dem in einem ersten Schritt die Probe mit einem Probenträger, der ein Mittel zur Abtötung unterschiedlicher Keime aufweist,das zumindest ein Antibiotikum und/oder zumindest ein Antimykotikum umfasst und mit einem Färbemittel, das ein Fluorophor und einen Quencher aufweist, das durch das Protein aufgespalten wird, so dass das Fluorophor freigesetzt wird, kontaktiert wird. In einem zweiten Schritt wird der Probenträger in eine Analyseeinrichtung eingebracht. In einem dritten Schritt werden von dem Fluorophor ausgehende Lichtemissionen von dem Probenträger durch die Analyseeinrichtung erfasst. In einem vierten Schritt wird eine Anzeige durch die Analyseeinrichtung ausgegeben, dass die Probe zumindest einen Keim enthält, der gegen das Mittel zur Abtötung unterschiedlicher Keime resistent ist, falls die Lichtemission einen Schwellwert übersteigt oder eine Anzeige ausgegeben, dass die Probe keine Keime enthält, die gegen das Mittel zur Abtötung unterschiedlicher Keime resistent ist, falls die Lichtemission den Schwellwert nicht überschreitet.

Vorteilhaft an diesem Verfahren ist, dass eine Detektion von gegen das Mittel zur Abtötung unterschiedlicher Keime resistenter Keime unabhängig von deren Klassifikation, wie etwa Art oder Stamm des Keimes, eine Aussage über eine Anwesenheit von multiresistenten Erregern im Allgemeinen und mithin ein möglicherweise erhöhtes Infektionsrisiko oder eine Erfordernis von gesonderten Maßnahmen, wie etwa eine Unterbringung des Patienten in einem Einzelzimmer oder die Beachtung besonderer Hygieneerfordernisse getroffen werden kann, ohne dass die Probe zeitaufwendig vorbereitet wurde

Bevorzugt wird der Probenträger beheizt, so dass eine Probe an oder in dem Probenträger ebenfalls beheizt ist und Inkubationsbedingungen, die eine Vermehrung von Keimen in der Probe beschleunigen, ausgesetzt ist.

Vorteilhaft an dieser Ausführungsform ist, dass basierend auf einer beschleunigten Vermehrung der Keime eine schnellere Aussage über ein Vorhandensein gegen das Mittel zur Abtötung unterschiedlicher Keime resistenter Keime getroffen werden kann.

Weitere, ausserhalb der beanspruchten Erfindung liegende Ausführungsbeispiele schaffen ein Verfahren, bei dem ein Färbemittel mit der Probe kontaktiert wird, so dass das Färbemittel in Keimen mit intakter Zellmembran eindringt und diese farblich markiert.

Vorteilhaft an diesem Verfahren ist, dass eine farbliche Unterscheidung eine verbesserte, d.h. frühere, Erkennung von resistenten Keimen ermöglicht. Ferner kann ein Maß für an von Erregern aufgenommenem Färbemittel ein Indikator für eine Menge von Erregern in der Probe sein und Aussagen zu einem Heilungsverlauf ermöglichen.

Allgemein wird ein Verfahren beschrieben, bei dem das Färbemittel einen Farbstoff aufweist, der von Stoffwechselprodukten lebender Keime aktiviert wird, so dass eine Erkennung von resistenten Keimen basierend auf der Farbstofferkennung und der Zellaktivität, d.h. des Stoffwechselproduktes, ermöglicht wird. Erfindungsgemäß handelt es sich bei den Stoffwechselprodukten um die Proteine DNase oder Hyaluronidase.

Vorteilhaft an diesem Verfahren ist, dass eine Erkennung der Stoffwechselprodukte resistenter Keime die resistenten Keime unverändert lassen kann, so dass die Keime für nachfolgende Untersuchungen zur Verfügung stehen können.

Die Erfindung betrifft außerdem eine Vorrichtung gemäß Anspruch 7 zur Durchführung des Verfahrens gemäß Anspruch 1.

Ist die Vorrichtung beispielweise ein mobiles Gerät, kann es sich für Aufgaben an variierenden Orten, wie etwa einer Patientenaufnahme in einem Krankenhaus eignen, so dass ein Untersuchungsergebnis bereits vor einer Stationszuweisung vorliegen kann.

Gemäß einer bevorzugten Ausführungsform weist die Analyseeinrichtung der Vorrichtung einen Foto-Multiplexer oder einen Ein-Photon-Detektor auf, um die Lichtemission von der Probe zu erfassen.

Vorteilhaft an diesem Ausführungsbeispiel ist, dass eine hochempfindliche Detektion der Lichtemission zu einer weiter beschleunigten Erkennung resistenter Keime führen kann.

Gemäß einer weiteren bevorzugten Ausführungsform umfasst die Vorrichtung eine

Beleuchtungseinrichtung zur Beleuchtung des Probenträgers und zur Anregung eines Farbstoffs eines Färbemittels zur Lichtemission, so dass die Lichtemission des Probenträgers auf der Beleuchtung durch die Beleuchtungseinrichtung basiert.

Die erfindungsgemäße Vorrichtung weist ein Behältnis auf, in welchem das Färbemittel angeordnet ist, wie etwa einen Tank.

Dadurch kann eine Zugabe von Färbemitteln innerhalb der ggf. verschlossenen Vorrichtung erfolgen und auf einen Benutzereingriff verzichtet werden.

Weitere vorteilhafte Ausführungsformen sind der Gegenstand der abhängigen Patentansprüche.

Bevorzugte Ausführungsbeispiele der vorliegenden Erfindung werden nachfolgend Bezug nehmend auf die beiliegenden Zeichnungen erläutert. Es zeigen:
- Fig. 1: ein schematisches Blockschaltbild einer Vorrichtung zur Erfassung resistenter Keime in einer Probe;
- Fig. 2: ein schematisches Blockschaltbild einer weiteren Vorrichtung zur Erfassung der resistenten Keime in der Probe mit einem gegenüber der Vorrichtung aus Fig. 1 vergrößerten Funktionsumfang;
- Fig. 3a: eine beispielhafte Ausführungsform eines Probenträgers, der als ein nahezu geschlossenes Volumen ausgeführt ist;
- Fig. 3b: eine schematische Darstellung eines Probenträgers, der als Platte ausgeführt ist.

Bevor nachfolgend Ausführungsbeispiele der vorliegenden Erfindung im Detail anhand der Zeichnungen näher erläutert werden, wird darauf hingewiesen, dass identische, funktionsgleiche oder gleichwirkende Elemente, Objekte und/oder Strukturen in den unterschiedlichen Figuren mit den gleichen Bezugszeichen versehen sind, so dass die in unterschiedlichen Ausführungsbeispielen dargestellte Beschreibung dieser Elemente untereinander austauschbar ist bzw. aufeinander angewendet werden kann.

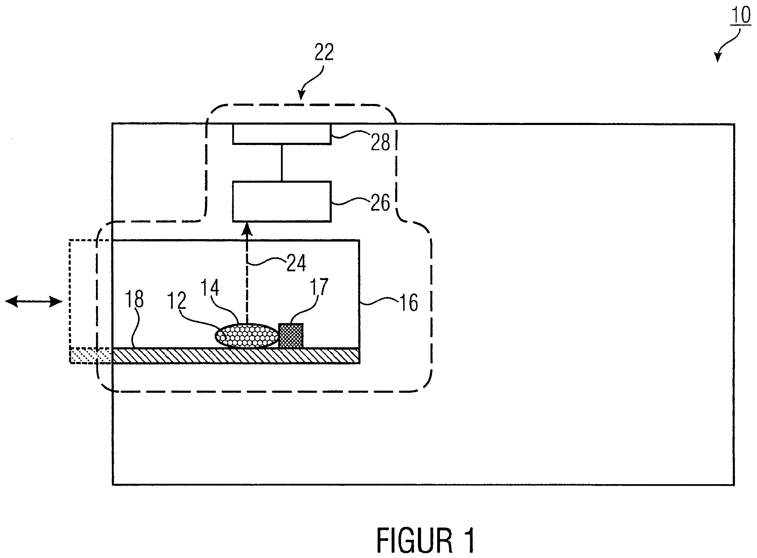

Fig. 1 zeigt ein schematisches Blockschaltbild einer Vorrichtung 10 zur Erfassung resistenter Keime 12 in einer Probe 14. Die resistenten Keime 12 sind als Punkte in der Probe 14 dargestellt. Die Vorrichtung 10 ist mit einem Probenträger 16 verbunden, der ein Nährmedium 17 und ein Mittel 18 zur Abtötung unterschiedlicher Keime aufweist. Die Probe 14, beispielsweise ein Nasen/Rachenabstrich eines Patienten, ist mit dem Mittel 18 kontaktiert. Die Probe 14 kann eine unbehandelte Patientenprobe sein, bspw. eine Probe, die direkt, etwa mittels eines Abstriches, einem Patienten entnommen und in oder an dem Probenträger 16 angeordnet wird. Alternativ kann die Probe 14 auch vorbehandelt werden, bspw. indem die Probe 14 mit dem Nährmedium 17 kontaktiert wird.

In anderen Worten wirkt das Mittel 18 auf Keime in der Probe 14 und tötet je nach Wirksamkeit des Mittels 18 Keime und/oder Erreger in der Probe 14, die nicht gegen das Mittel 18 resistent sind. Keime 12, die weiterhin Stoffwechsel betreiben, können als gegen das Mittel 18 resistent bezeichnet werden. In anderen Worten können die resistenten bzw. multiresistenten Keime 12 einen Antibiotikamix überlebende Bakterien sein.

Bei dem Mittel 18 kann es sich um ein Antibiotikum, eine Kombination mehrerer Antibiotika, ein Antimykotikum, eine Mischung oder Kombination mehrerer Antimykotika oder eine Mischung aus einem oder mehreren Antibiotika und Antimykotika handeln. Das Mittel 18 kann somit eine individuelle Kombination an Wirkstoffen sein, deren Wirksamkeit gegenüber Keimen in der Probe 14 getestet werden soll. Die Wirkstoffe können jeweils geeignet sein, um Keime einer Art oder eines Stammes abzutöten, so dass gegen das Mittel 18 resistente Keime gegen zwei oder mehr Wirkstoffe resistent sind und als multiresistent bezeichnet werden können. Das Mittel 18 kann ein Feststoff, ein Gas oder eine Flüssigkeit sein. Das Nährmedium 17 ist ausgebildet, einen Nährstoff für die Keime in der Probe 14 bereitzustellen, um deren Wachstum und Zellteilung zu ermöglichen. Bei dem Nährmedium 17 kann es sich bspw. um Agar oder eine Nährbuillon handeln. Bei den Keimen kann es sich um Bakterien oder Pilzerreger handeln.

Vorrichtung 10 umfasst eine Analyseeinrichtung 22, die ausgebildet ist, um Lichtemissionen 24 von dem Probenträger 16 zu erfassen. Der Probenträger kann ganz oder teilweise transparent oder zumindest für einen Wellenlängenbereich der Lichtemissionen 24 teilweise transparent ausgestaltet sein, so dass die Lichtemission 24, wenn sie von der Probe 14 ausgeht, ein Material, aus welchem der Probenträger 16 gebildet ist, zumindest teilweise durchdringen und zur Analyseeinrichtung 22 gelangen kann.

Die Analyseeinrichtung 22 weist eine Detektionseinrichtung 26 auf, die ausgebildet ist, um die Lichtemissionen 24 zu empfangen. Die Analyseeinrichtung 22 weist ferner eine Anzeigeeinrichtung 28 auf, die ausgebildet ist, um anzuzeigen, ob von der Detektionseinrichtung 26 Lichtemissionen 24 empfangen worden sind. In anderen Worten ist die Analyseeinrichtung 22 ausgebildet, um die Lichtemissionen 24 zu erfassen, wenn multiresistente Keime 12 in dem Probenträger angeordnet sind. Alternativ erfasst der Detektor 26 bspw. keine Lichtemission 24, wenn keine multiresistenten Keime 24 in der Probe 14 angeordnet sind.

Die Lichtemissionen 24 können beispielsweise auf einer Phosphoreszenz, einer Fluoreszenz oder einer Lumineszenz bzw. aus einer Mischung dergleichen in Keimen der Probe 14 resultieren oder durch Faktoren bewirkt werden, die die Keime der Probe 14 produzieren und abgeben. Sind durch das Mittel 18 alle nicht resistenten Keime abgetötet, kann aus einer empfangenen Lichtemission 24 geschlossen werden, dass die Probe 14 gegen das Mittel 18 resistente Keime 12 aufweist. Ist das Mittel 18 ausgebildet, um verschiedene, unterschiedliche Keime abzutöten, so ist die Vorrichtung 10 ausgebildet, um mit der Analyseeinrichtung 22 die Anwesenheit von multiresistenten Keimen 12 in der Probe 14 anzuzeigen. Die Lichtemission kann auf der Implementierung einer Auswerte- oder Nachweisstrategie basieren. Verschiedene mögliche Nachweisstrategien werden weiter unten erläutert. Erfindungsgemäß basiert die Lichtemission auf einer Fluoreszenzemission eines Fluorophors, das von einem dieses Fluorophor und einen Quencher enthaltenden Färbemittel durch Enzymaktivität von von lebenden Keimen gebildeter DNase oder Hyaluronidase abgespalten wird.

Die Analyseeinrichtung 22 ist ausgebildet, um eine Anzeige auszugeben, dass die Probe keine Keime enthält, die gegen das Mittel 18 zur Abtötung unterschiedlicher Keime resistent sind. Beispielsweise kann die Analyseeinrichtung 22 ausgebildet sein, um eine Menge vom Detektor 26 empfangener Lichtemission 24 mit einem Schwellwert zu vergleichen. Wird von dem Detektor 26 ein höheres Maß an Lichtemission 24 empfangen als es der Schwellwert angibt, so kann eine entsprechende Anzeige an der Anzeigeeinrichtung 28 ausgegeben werden. Die Anzeigeeinrichtung 28 kann alternativ oder zusätzlich so lange anzeigen, dass keine Keime, die gegen das Mittel 18 resistent sind, in der Probe 14 enthalten sind, bis der Umfang der Lichtemission 24 den Schwellwert übersteigt. Ein Schwellwert kann bspw. einen Empfang von Umgebungslicht (Fehlerlicht) an dem Detektor 26 kompensieren, bei dem der Detektor 26 eine Lichtemission empfängt, die nicht von der Probe 14 ausgeht.

Die Anzeigeeinrichtung 28 kann beispielsweise ein Display, ein Monitor, eine Statusleuchte oder Status-LED oder eine beliebige andere Einrichtung zur Darstellung von Information sein. Alternativ oder zusätzlich kann die Anzeige, die von der Anzeigeeinrichtung 28 ausgegeben wird, auch ein akustisches oder haptisches (bspw. eine Bewegung oder eine Vibration) Signal sein.

Der Detektor 26 kann beispielsweise einen Foto-Multiplexer oder einen Ein-Photon-Detektor umfassen, der ausgebildet ist, um die Lichtemission 24 bereits in einem Umfang von einem oder wenigen Photonen zu erfassen. In diesem Fall kann der Schwellwert beispielsweise einen Wert von 0 aufweisen. In anderen Worten ermöglicht der Ein-Photon-Detektor eine hochauflösende Messung.

Vorrichtung 10 kann beispielsweise so verwendet werden, dass die Probe 14 mit dem Probenträger 16 kontaktiert wird. Der Probenträger 16 kann eine Küvette, ein anderes Gefäß oder ein sonstiger Körper sein, mit dem oder in dem die Probe 14 mit dem Probenträger 16 kontaktiert werden kann. Beispielsweise kann die Probe durch einen Einspülvorgang mit dem Probenträger 16 kontaktiert werden. Der Probenträger 16 wird in die Vorrichtung 10 bzw. die Analyseeinrichtung 22 eingebracht. Dies kann beispielsweise durch einen Steck- oder Schraubvorgang, einen Klemmvorgang oder einer sonstigen Methode zur Verbindung des Probenträgers 16 mit der Analyseeinrichtung 22 erfolgen, so dass der Probenträger 16 bezüglich der Vorrichtung 10 entfern- und/oder austauschbar ist. Alternativ kann der Probenträger 16 auch fest mit der Vorrichtung 10 verbunden sein.

Vorrichtung 10 ermöglicht beispielsweise bei einer Patientenaufnahme durch einen Schnelltest einen Hinweis darauf zu erhalten, ob eine Isolierung und/oder eine Einweisung in eine Quarantänestation für den Patienten, von dem die Probe 14 stammt, erforderlich ist.

Vorteilhaft ist, dass eine Vorbehandlung der Patientenprobe, beispielsweise durch kulturelle Anzucht der in ihr enthaltenen Erreger und anschließender Keimisolation zur Züchtung von Reinkulturen von Keimen, mit denen im Anschluss daran ein Antibiogramm durch Ausstreichen der Keime auf einem festen Nährmedium (Agar) und anschließendem Auflegen von Antibiotika-Plättchen erstellt wird, entfallen kann.

Zusätzlich kann eine Probenvorbereitung zur Zerstörung von Zellmembranen zur Erlangung der DNA-Stränge, wie es beispielsweise für die PCR erforderlich ist, entfallen. In anderen Worten kann eine komplette, unbehandelte Patientenprobe in die Vorrichtung oder das Testverfahren eingegeben werden.

Für eine Ableitung von solchen Maßnahmen kann zu diesem Zeitpunkt ggf. auf eine Bestimmung eines Typs, einer Art oder eines Stammes der multiresistenten Keime 12 verzichtet werden, da manche zu ergreifenden Maßnahmen wie etwa eine Unterbringung auf einer Isolierstation und/oder Quarantänestation unabhängig vom genauen Typ, Art oder Stamm des multiresistenten Erregers sein können. Eine durch den Verzicht der genauen Kategorisierung der multiresistenten Keime 12 erzielte Zeitersparnis die zu einer unspezifischen "Ja/Nein"-Aussage über eine Anwesenheit multiresistenter Keime führt, kann große Vorteile bezüglich der Patientenversorgung und/oder des Schutzes anderer Patienten vor einer Infektion mit multiresistenten Keimen ermöglichen, da die entsprechenden Maßnahmen wesentlich schneller ergriffen werden können. Das schnellere Ergreifen der Maßnahmen kann als ein erster Zeitvorteil beschrieben werden. Weist die Analyseeinrichtung 22 einen hochempfindlichen Silizium-Fotomultiplexer oder einen Ein-Photon-Detektor auf, so kann bereits eine geringe Anzahl, möglicherweise bereits ein einzelner resistenter Keim 12, in der Probe 14 erkannt werden, so dass auf eine lange Inkubationszeit, bis eine gewisse, ggf. hohe Anzahl von multiresistenten Keimen 12 in der Probe 14 herangewachsen ist, verzichtet werden, was zu einem zweiten Zeitvorteil führen kann. In anderen Worten kann durch Einsatz eines hochempfindlichen Silizium-Fotomultipliers geringere Konzentrationen an Erregern nachgewiesen werden, als bisher üblich. Dadurch ist es möglich, lange Inkubationszeiten der derzeit üblichen Methoden zur mikrobiologisch-kulturellen Anzucht der Erreger zu umgehen.

In anderen Worten kann die Patientenprobe (z.B. Nasen-Rachenabstrich) in ein Gefäß, d. h. Probenträger, welches Universal-Nährmedium und ggf. Nachweisreagenzien enthalten kann, eingespült werden. Damit nur multiresistente Keime bei diesem Nachweis erfasst werden, wird das Nährmedium zusätzlich mit geeigneten Antibiotikamix und ggf. Antimykotika versetzt. Der Behälter kann so ausgelegt sein, dass ein mit Probematerial beladener Abstrichtupfer eingetaucht und ausgespült werden kann. Eine Verengung am Verschluss des Messgefäßes, welches für eine optische Detektion des Messsignals ausgelegt ist, ermöglicht ein gleichmäßiges Abstreifen des Probenehmers. Nach Einbringen des zu untersuchenden Probematerials in das Medium wird der Behälter mit einem Deckel verschlossen und unter optimalen Bedingungen inkubiert. Alle Erreger, die keine Resistenz gegen die eingesetzten Antibiotika aufweisen, stellen ihren Stoffwechsel ein und sterben. Alle resistenten Keime werden sich unter den Inkubationsbedingungen vermehren.

Anschließend werden überlebende multiresistente Erreger durch eine fluoreszenzbasierte Reaktion oder Farbreaktion nachgewiesen. Die verwendeten Farbstoffe können in Lösung oder auch an partikuläre Systeme (z.B. Mikro- oder Nanopartikel) gebunden sein. Ebenfalls ist eine Immobilisierung der Farbstoffe an eine feste Phase - sofern es die verwendete Nachweisstrategie vorsieht - denkbar. Der Schnelltest kann in einem Gerät, etwa in Form eines Handgerätes durchgeführt werden, welches beheizbar ist, ein angemessenes Schütteln der Probe erlaubt und mit Elementen für spektroskopische Untersuchung der Probe ausgelegt ist.

Das durchgeführte Verfahren kann auch als ein MRE-Test oder Schnelltest zur Vor-Ort-Erkennung multiresistenter Erreger bezeichnet werden. Der MRE-Test kann im Vergleich zu den derzeit verwendeten Nachweismethoden, darunter hauptsächlich mikrobiologisch kulturelle Anzucht und PCR, auch von nicht speziell für derartige Tests geschultem Personal, beispielsweise Krankenpfleger, Krankenschwestern, etc. durchgeführt werden, was eine hohe Verfügbarkeit der Tests ermöglichen kann. Hochspezifische Nachweisemethoden werden für gewöhnlich in speziell dafür ausgestatteten Labors ausgeführt, die oft an einem anderen Ort als die jeweilige Arztpraxis und/oder die jeweilige Klinik mit den zu untersuchenden Patienten sind. Ferner kann eine Fehlerquote des Tests reduziert werden, was trotz nicht geschultem Personal eine zuverlässige Erkennung multiresistenter Erreger ermöglicht. Der MRE-Test kann, auch weil bei einer Ausführung der Vorrichtung als Handgerät auf einen Einsatz von stationärem Laborequippment verzichtet werden kann, kostengünstiger und kleinbauender ausgeführt sein und in wesentlich kürzerer Zeit anzeigen, ob multiresistente Erreger zugegen sind. Alternativ kann Vorrichtung 10 auch als eine stationäre Apparatur ausgeführt sein, um bspw. mehrere Tests, wie etwa einen Schnelltest und zusätzlich einen Test zur Typisierung der MRE-Erreger durchzuführen.

In anderen Worten kann eine Anwendung des MRE-Schnelltest zum Nachweis multiresistenter Erreger bzw. zur Kontrolle des Therapieverlaufs in nahezu jeder Funktionsstelle des medizinischen Bereiches, wie etwa eines Krankenhauses oder einer Facharztpraxis, erfolgen. Auch auf anderen medizinischen Feldern und in anderen Bereichen des täglichen Lebens ist eine Anwendung vorstellbar, beispielsweise bei Lebensmittelkontrollen. Im Bereich der medizinischen Anwendung bieten dermatologische Analyseverfahren von Pilzen, Bakterien und anderen Keimen auf der Haut ein weiteres Anwendungsfeld. Vorrichtung 10 kann als Handgerät ausgeführt und zur Vor-Ort-Diagnostik eingesetzt werden. Vorrichtung 10 kann so ausgeführt sein, dass sie einfach zu handhaben und preiswert in der Herstellung ist. Ein weiterer Vorteil ist, dass multiresistente Erreger keimunspezifisch nachgewiesen werden können und sich der Nachweis nicht nur auf eine Spezies beschränkt. Mittels derzeit durchgeführter PCR wird das Vorhandensein MRSA-spezifischer DNA-Sequenzen, wie etwa das SCCmec-Gen, nachgewiesen. Dieser Test ist daher sehr keimspezifisch und dauert in etwa 2,5 Stunden. Der Test ist vergleichsweise teuer und ungeeignet zur Vor-Ort-Erkennung, wie es in [4, 5] beschrieben ist.

Ferner werden in derzeitigen Tests nicht alle Bakterien oder Pilzerreger erfasst, die Multiresistenzen erworben haben, deren Vorhandensein jedoch eine Isolation des Patienten im Sinne einer Kontaminationsprävention erfordern würde. Demgegenüber erlaubt eine individuelle, ggf. über die Zeit variierende, Zusammenstellung des Mittels 18 eine beliebige Zusammenstellung von Wirkstoffen, die nahezu jeden beliebigen Keim abtöten sollten. Wird ein Keim von einem derartigen Mittel nicht abgetötet, können auch neue, unbekannte oder seltene multiresistente Erreger nachgewiesen werden.

Das mit Vorrichtung 10 implementierte Verfahren erlaubt eine parallele Durchführung mehrerer Tests. Das Probematerial kann in unterschiedliche Probegefäße, die jeweils verschiedene Antibiotika im Nährmedium enthalten, in ein MRE-Test-Systems mit mehreren Analyseeinrichtungen oder in mehrerer Vorrichtungen eingebracht werden, so dass durch den Schnelltest auch die für die Behandlung passenden Antibiotika, also jene die einen Großteil oder alle der Keime abtöten, ermittelt werden können. So können jeweils verschiedene Antibiotika und/oder Antimykotika in den Probenträgern angeordnet sein. Dort, wo bestimmte Keime absterben, kann auf eine entsprechende Therapiemöglichkeit geschlossen werden. Dies kann sich aufgrund des Zeitfaktors positiv auf den Therapieverlauf der Infektion auswirken, da mehrere parallel durchgeführte Tests, die Zeitvorteile des Schnelltests beibehalten. Das bedeutet, dass durch eine parallele Ausführung mehrerer Schnelltests auch eine Bestimmung des multiresistenten Keimes indirekt erfolgen kann, indem überprüft wird, welcher Wirkstoff bzw. Antibiotika-und/oder Antimykotika-Mix sämtliche oder nahezu alle Keime abtötet. Die Analyseeinrichtung 22 kann ausgebildet sein, um einen oder mehrere mögliche Erregertyp auszugeben.

In anderen Worten kann Vorrichtung 10 ein Schnelltest zum MRE-Nachweis als Eingangsuntersuchung eingesetzt werden. Dieser Test kann das Übertragungsrisiko der Erreger auf andere Patienten deutlich minimieren.

Alternative Ausführungsbeispiele zeigen Vorrichtungen bei denen die Analyseeinrichtung 22 ausgebildet ist, um ein Maß der Lichtemission 24, das ein Maß für eine Konzentration oder eine Menge von multiresistenten Keimen 12 in der Probe 14 sein kann, relativ, skaliert oder absolut darzustellen, so dass beispielsweise eine Vermehrung oder Abnahme der multiresistenten Keime 12 in dem Probenträger 16 mittels eines über verschiedene Zeitpunkte variierenden Anzeigewert in der Anzeigeeinrichtung 28 dargestellt wird.

Obwohl der Detektor 26 so beschrieben wurde, dass er einen Fotomultiplier oder Ein-Photon-Detektor aufweist, können alternativ oder zusätzlich auch andere Detektorelemente, bspw. mit miniaturisierten Siliziumbauelementen, angeordnet sein. Siliziumbauelemente können einen geringen Energieverbrauch aufweisen und zugleich kostengünstig sein. Ferner kann der Detektor 26 auch als Empfänger eines Konfokalmikroskops ausgeführt sein. Ein Einsatz eines Bauteils, welches in der Lage ist, sogar einzelne Photonen nachzuweisen, kann eine Verkürzung der Nachweismethode auf wenige Stunden ermöglichen. (Eine Testzeit kann beispielsweise weniger als 5 Stunden, weniger als 3 Stunden oder weniger als 1 Stunde betragen) Eine mehrstündige Kultivierung zur Vervielfachung der Probenmengen bzw. der darin enthaltenen Keime kann dann unnötig sein.

Obwohl das Mittel 18 und das Nährmedium 17 als getrennte Objekte dargestellt sind, können das Nährmedium 17 und/oder das Mittel 18 flüssige oder gelartige Substanzen sein, die heterogen oder homogen mit einander vermischt sind. Die Vermischung kann bspw. durch einen Schüttel- oder Rührprozess erfolgen. Alternativ kann das Mittel 18 auch in dem Nährmedium 17 gelöst sein oder anders herum, so dass das Mittel 18 oder das Nährmedium 17 eine Dispersion oder Suspension in dem jeweils anderen Bestandteil ist. Ferner kann die Probe 14 in dem Mittel 18 und/oder dem Nährmedium 17 gelöst oder mit dem Mittel 18 und/oder dem Nährmedium 17 heterogen oder homogen verbunden sein, bspw. wenn die Probe 14 in dem Mittel 18 und/oder dem Nährmedium 17 eingewaschen oder eingerührt ist, wenn das Mittel 18 und/oder das Nährmedium 17 flüssig ist.

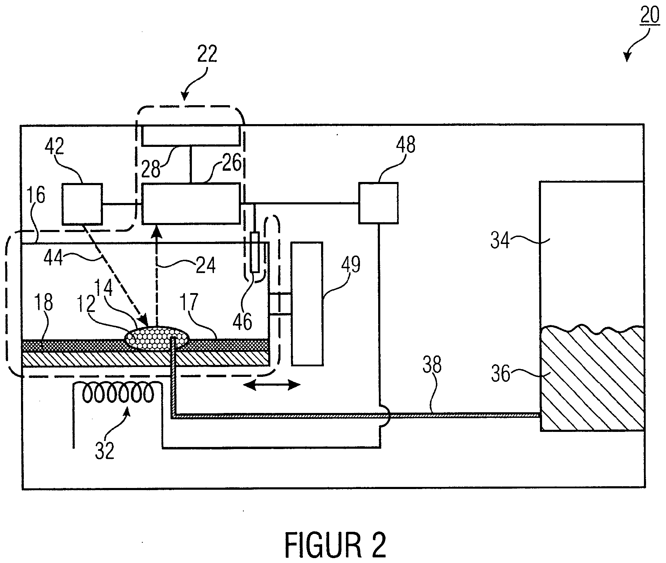

Fig. 2 zeigt ein schematisches Blockschaltbild einer weiteren Vorrichtung 20 zur Erfassung der resistenten Keime 12 in der Probe 14. Vorrichtung 20 weist einen gegenüber Vorrichtung 10 vergrößerten Funktionsumfang auf.

Vorrichtung 20 umfasst ein Behältnis 34, das ausgebildet ist, um ein Färbemittel 36 zu beinhalten und das Färbemittel 36 über eine Zuleitung 38 mit der Probe 14 zu kontaktieren. Das Färbemittel 36 ist ausgebildet, um in die resistenten Keime 12 ganz oder teilweise einzudringen und mithin zu markieren oder, um basierend auf einem Stoffwechselprodukt der resistenten Keime 12 eine Farbreaktion zu zeigen, so dass die Lichtemission 24 von dem Farbstoff bzw. dem Färbemittel 36 beeinflusst ist. In anderen Worten können resistente 12 Keime für einen direkten Nachweis markiert oder Reaktionsprodukte der Stoffe der resistenten (lebenden) Keime 12 in dem Probenträger messbar gemacht werden, so dass ein indirekter Nachweis der resistenten Keime 12 ermöglicht ist. Das Färbemittel 36 kann beispielsweise Fluoreszenz- oder Phosphoreszenzstoffe aufweisen.

Vorrichtung 20 weist ferner eine Beleuchtungseinrichtung 42 auf, die ausgebildet ist, um eine Beleuchtungsstrahlung 44 auszusenden, so dass die Beleuchtungsstrahlung 44 die Probe 14 in dem oder an dem Probenträger 16 beleuchtet. Die Beleuchtungsstrahlung 44 kann eine elektromagnetische Strahlung und bspw. ausgebildet sein, um eine Fluoreszenz oder eine Phosphoreszenz eines Farbstoffs des Färbemittels 36 anzuregen. Der Probenträger 16 ist für die Beleuchtungsstrahlung 44 und die Lichtemission 24 zumindest teilweise transparent so dass die Beleuchtungsstrahlung den Probenträger 16 in eine Richtung zur Probe 14 mit und die Lichtemission 24 den Probenträger in eine Richtung von der Probe 14 zu dem Detektor 26 zumindest teilweise durchdringen kann.

Eine Ausprägung oder Ausgestaltung des Färbemittels 36 kann auf einer Auswertestrategie des Testverfahrens basieren oder davon abhängen. Verschiedene Auswertestrategien werden im Folgenden erörtert. Gemäß einem nicht beanspruchten Beispiel kann das Färbemittel 36 beispielsweise ausgebildet sein, um durch Zellmembranen von Keimen, die den Kontakt mit dem Mittel 18 überlebt haben, einzudringen und so die lebenden, d. h. resistenten Keime 12 in dem Probenträger 16 zu markieren. Alternativ ist ebenfalls vorstellbar, dass das Färbemittel 36 eine Farbänderung oder eine Lichtänderung basierend auf einem Stoffwechselprodukt der resistenten Keime 12 anzeigt, indem die Farbreaktion oder die Lichtänderreaktion, wie etwa eine Fluoreszenz, eine Phosphoreszenz oder eine Lumineszenz von einem Stoffwechselprodukt der multiresistenten Keime 12 induziert oder katalysiert wird, so dass ein Farbstoff, wie etwa ein fluoreszenter oder phosphoreszenter Stoff, aktiviert oder freigesetzt wird. In anderen Worten kann das Bakterium selbst oder einer von ihm gebildeten Produkte/Faktoren (Proteine) nachgewiesen werden.

Gemäß einem nicht beanspruchten Beispiel kann das Färbemittel bspw. ausgebildet sein, um die resistenten Keime 12 basierend auf einer Membranintegrität der resistenten Keime 12 zu markieren, und so den Nachweis zu ermöglichen.

Eine mögliche Strategie gemäß einem nicht beanspruchten Beispiel umfasst eine farbliche Markierung von lebenden Keimen. Die Einfärbung kann bspw. auf einer Einfärbung der Nukleinsäuren in den Zellen erfolgen, so dass eine Auswertung der Lichtemission 24 basierend auf einer Menge oder Intensität der Lichtemission 24 bezüglich einer Wellenlänge der Einfärbung erfolgen kann.

Zusätzlich umfasst eine weitere mögliche Strategie gemäß einem nicht beanspruchten Beispiel eine farblich unterschiedliche Markierung von lebenden und toten Keimen beispielsweise durch eine Kontaktierung zweier farblich unterschiedliche Farbstoffe bzw. Färbemittel mit der Probe 14. Ein erstes Färbemittel, beispielsweise ein Farbstoff mit grüner Fluoreszenz, ist ausgebildet, um intakte und defekte Zellmembranen, d.h. Zellmembranen von lebenden und toten Keimen, zu durchdringen. In anderen Worten werden lebende und tote Keime mit einer ersten Farbe (grün) markiert. Ein zweites Färbemittel, beispielsweise ein Farbstoff mit roter Fluoreszenz, ist ausgebildet, um lediglich defekte Membranen, d.h. Membranen von toten Keimen, zu passieren. In anderen Worten werden tote Keime mit einer zweiten Farbe (rot) markiert. Werden beide Färbemittel in einem bestimmten Verhältnis eingesetzt, so können beispielsweise lebende Keime grün erscheinen, während tote Keime rot erscheinen. Die Auswertung der Auswerteeinrichtung 22 kann bei einer farblichen Unterscheidung lebender und toter Keime mit optischen Kriterien, beispielsweise über einen Fluoreszenz-Reader, erfolgen, wie es beispielsweise in [9, 10, 11] beschrieben ist. Das bestimmte Verhältnis kann von der Ausgestaltung oder einer Empfindlichkeit, bspw. einer Farbempfindlichkeit, der Analyseeinrichtung und/oder von einem gewünschten Farbkontrast abhängen.

Gemäß einer weiteren Strategie gemäß einem nicht beanspruchten Beispiel kann eine Identifikation lebender Keime über eine Messung einer Aktivität von zellularer Esterase erfolgen. Die Probe 14 kann mit zwei unterschiedliche Fluoreszenzfarbstoffen kontaktiert werden, bspw. indem die Fluoreszenzfarbstoffe in das Nährmedium 17 zugesetzt oder über die Zuleitung 38 in den oder an den Probenträger 16 geführt werden. Ein erster Farbstoff kann beispielsweise ein Acetomethyl (AM)-Ester-basiertes Substrat, wie etwa Calcein-AM, 2',7'-bis-(2-carboxyethyl)-5-(and-6)-carboxyfluorescein (BCECF)-AM, ChemChrome V6 (CV6) oder das Fluorescein-Diacetat FDA sein. Ein zweiter Farbstoff kann bspw. ein Nukleinsäure-färbendes Fluorophor, wie etwa Ethidium-Homodimer-I bzw. III oder CSE/2 in dem Färbemittel 36 angeordnet sein.

Acetomethyl-Ester-basierte Substrate sind in der Regel membrangängig, d. h. die Substrate werden von den resistenten Keimen 12 aufgenommen, und sind nicht-fluoreszent. Im Inneren des Keims werden die Acetomethyl-Reste enzymatisch durch unspezifische Esterasen abgespalten. Das führt zur Fluoreszenz des verbleibenden Fluorophors. Die Substanz, d. h. der Fluorophor, verbleibt in der Zelle. In anderen Worten können an einen Fluorophor, wie etwa Fluorescein über eine Esterbindung Acetomethyl-Reste angebunden werden. Der Fluorophor allein ist oft nicht membrangängig, durch Einführen der AM-Reste kann er jedoch die Zellmembran passieren. Im Zytoplasma der Zelle befinden sich Esterasen, die diese Esterbindungen des modifizierten Fluorophors spalten. Zurück bleibt der Fluorophor ohne AM - in dieser Form ist er fluoreszent und nicht mehr membrangängig, kann die Zelle also auch nicht mehr verlassen. Bei Fluorescein leuchtet die Zelle bei entsprechender Anregung grün. So wird gewährleistet, dass lediglich lebende Zellen der Keime 12 erfasst werden, da Membranen toter Keime inaktiv bzw. zerstört sind. Durch einen weiteren Zusatz eines DNA-bindenden Fluorophors, wie beispielsweise Ethidium-Homodimer-I oder III, der nur defekte Membranen passieren kann, ist es möglich, lebende und tote Bakterien unterschiedlich fluoreszent zu markieren, wie es beispielsweise durch [12, 13, 14, 15] beschrieben ist.

Ein Hinzufügen eines zweiten Färbemittels, das tote Bakterien oder Keime markiert, kann eine Auflösung des Analyseverfahrens erhöhen.

Gemäß einer weiteren Auswertestrategie gemäß einem Ausführungsbeispiel kann ein Nachweis von multiresistenten Keimen 12, über von den resistenten Keimen 12 gebildete Proteine geführt werden. Als ein Virulenzfaktor, der ein Maß für eine gesundheitliche Gefährdung und/oder ein Ansteckungsrisiko sein kann, kann sich beispielsweise das Exoenzym DNase eignen, wenn eine Probe auf eine Anwesenheit multiresistenter Keime vom Typ Staphylococcus aureus analysiert wird. Das Enzym DNase wird vom lebenden Keim gebildet (exprimiert), und so an seine Umgebung, bspw. in das Nährmedium 17, das Mittel 18 und/oder den Farbstoff 36 abgegeben. Der Nachweis von DNase kann durch einen Zusatz bzw. eine Kontaktierung kurzer, bspw. mit einer Anzahl zwischen 20 und 200, zwischen 30 und 150 oder zwischen 40 und 60 Basenpaaren, bspw. 50, gelabelter DNA-Fragmente mit der Probe 14 erfolgen. In anderen Worten erfolgt die Kontaktierung des Farbstoffs in der Umgebung, in die das Exoenzym abgegeben wird, wie etwa das Nährmedium 17, in dem der Keim auch angezüchtet werden soll. Die gelabelten DNA-Fragmente können synthetisch hergestellt und ein Fluoreszenzlabel und einen Quencher aufweisen.

Die DNA-Fragmente können nach Hybridisierung von drei Einzelsträngen, einem ungelableten (z.B. aus 50 Nukleotiden bestehend) und zwei Oligonukleotiden, die dem ungelableten Einzelstrang komplementär sind, entstehen. Eines der beiden Oligonukleotide ist bspw. mit einem Quencher versehen, das andere mit einem Fluorophor, und zwar so, dass sich Fluorophor und Quencher nach Hybridisierung der drei Einzelstränge räumlich so nahe kommen, dass die Fluoreszenz gequencht wird. Bei einer Reaktion mit dem Exoenzym DNase wird die DNA gespalten, Flourophor und Quencher können sich räumlich von einander entfernen, so dass die Fluoreszenz wird nicht mehr unterdrückt und der Fluorophor wird nachweisbar wird. Der Quencher ist ausgebildet, um eine Fluoreszenz des Fluorophors zu quenchen, d. h. zu unterdrücken.

Eine Anwesenheit von DNase führt zu einer Spaltung des DNA-Fragments, so dass an einem Teil des Fragments der Fluorophor und an einem anderen Teil der Quencher angeordnet ist. Der Fluorophor kann zu einer Fluoreszenz unabhängig von dem Quencher angeregt werden, da der Quencher auf einem anderen Teil angeordnet ist. In anderen Worten wird der Quencher, d.h. der Unterdrücker der Fluoreszenz, abgespalten, so dass die Fluoreszenz an dem entsprechenden Segment nicht mehr unterdrückt ist. Nach Anregung des Fluorophors, beispielsweise durch die Beleuchtungseinrichtung 42 und die Beleuchtungsstrahlung 44 kann eine Fluoreszenz in dem Nährmedium, in dem sich zumindest ein multiresistenter Keim 12 befindet, angeregt und von dem Detektor 26 erfasst werden. Ein Nachweis der Aufspaltung der DNA-Fragmente ist beispielsweise in [16, 17] beschrieben.

Alternativ ist auch ein Nachweis anderer Exoenzyme möglich. Beispielsweise wird in Übereinstimmung mit Ausführungsbeispielen das Enzym Hyaluronidase von lebenden resistenten Keimen 12, bspw. von Bakterien wie Staphylococcus aureus, Staphylococcus epidermidis, Streptococcus pyogenes exprimiert und an seine Umgebung abgegeben. Ein Nachweis der Hyaluronidase kann durch Zusatz kurzer, gelabelter Hyaluronsäure-Fragmente in die Umgebung der resistenten Keime 12, etwa in das Nährmedium 17, erfolgen. Die Hyaluronsäure-Fragmente können an einem Ende mit einem Quencher und am anderen Ende mit einem Fluorophor versehen sein. Dadurch ist die Fluoreszenz ebenfalls gequencht. Ist Hyaluronidase zugegen, so kommt es zur Spaltung des Fragmentes und nach entsprechender Anregung kann eine Fluoreszenz detektiert werden, wie es beispielsweise in [18, 19, 20] beschrieben ist.

Gemäß einer weiteren Strategie gemäß einem nicht beanspruchten Beispiel wird ein Zuckerabbau in der Umgebung der multiresistenten Keime 12 durch selbige, nachgewiesen. Das Enzym Galaktosidase spaltet beispielsweise Laktose in Glukose und Galaktose. Ist an D-Galaktose, ein Zuckermolekül, welches sich strukturell von der Glukose nur gering unterscheidet, anstelle von Glukose ein Fluorophor gebunden, welcher nur dann fluoresziert, wenn er frei in Lösung ist oder welcher seine Fluoreszenzeigenschaften, wie Intensität oder Wellenlänge bzw. Farbe, ändert, sobald er nicht mehr an D-Galaktose gebunden ist, kann Zuckerspaltung sichtbar gemacht werden, wenn das Enzym Galaktosidase den Fluorophor von D-Galaktose abgespalten hat.

Sind multiresistente Keime 12, die Galaktosidase produzieren, bspw. Staphylococcus aureus oder coliforme Bakterien, und den gelabelten Zucker (D-Galaktose, welche mit dem Fluorophor gelabelt ist) im Medium, bspw. ein Gemisch aus Probe 14, Mittel 18, Nährmedium 17 und/oder Färbemittel 36, enthalten, kann Zuckerspaltung und mithin die Anwesenheit der multiresistenten Keime 12 sichtbar gemacht werden, wenn die D-Galaktose abgespalten und ein Fluoreszenzsignal des Fluorophors von der Analyseeinrichtung 22 detektiert wird. Als Fluorophor kann sich beispielsweise Fluorescein eignen, welches als Acetylfluorescein nicht fluoreszent ist, aber nach der durch das Enzym Galaktosidase katalysierten Hydrolyse zur Abspaltung des Zuckerrestes in ein fluoreszentes Produkt umgewandelt wird, wie es beispielsweise in [21, 22] beschrieben ist.

Gemäß einer weiteren Nachweisstrategie gemäß einem nicht beanspruchten Beispiel können überlebende respiratorisch aktive, d.h. Sauerstoff oder ein anderes Atmungsgas umsetzende, resistente Keime 12 auch mit einer Redox-Probe nachgewiesen werden. Hierfür kann die Probe 14 beispielsweise mit einem 5-Cyano-2,3-Ditolyl Tetracolium Chloride (CTC) kontaktiert werden. Oxidiertes CTC ist farblos und nicht-fluoreszent. Diese Substanz wird in die Zelle des lebenden multiresistenten Keimes 12 aufgenommen. Intrazellulär fungiert es in der Elektronentransportkette (Atmungskette) als künstlicher, d.h. weiterer, Elektronenakzeptor und wird bei dem Atmungsvorgang während der Atmungskette reduziert. Reduziertes CTC ist fluoreszent, fällt als unlösliches CTC-Formazan aus und akkumuliert in lebenden Zellen der multiresistenten Keime 12, wie es in [23] beschrieben ist.

Die verschiedenen dargestellten Nachweisstrategien sind lediglich exemplarisch zu verstehen. Prinzipiell können auch andere Nachweisstrategien zum Nachweis multiresistenter Keime 12 angewendet werden. Die verschiedenen Nachweisstrategien können so miteinander kombiniert werden, dass ein spezifischer Erregernachweis über dessen erregerspezifische Enzymausstattung ermöglicht wird, beispielsweise durch eine sequenzielle Ausführung mehrerer Tests oder durch eine parallele Ausführung mehrerer Tests in mehreren Vorrichtungen.

Die beschriebenen Strategien zur Erkennung von multiresistenten Keimen 12 basieren auf dem Nachweis metabolisch aktiver Keime oder Bakterien. Damit sind diese Methoden auch geeignet zum Einsatz in der Therapieverlaufskontrolle. Dies kann beispielsweise derart erfolgen, dass in verschiedenen Zeitabständen von beispielsweise Stunden, Tagen oder Wochen Verfahren zur Erfassung multiresistenter Keime 12 durchgeführt werden und eine Konzentration von multiresistenten Keimen 12 in der jeweiligen Probe 14 angezeigt wird. Weiterhin ist denkbar, dass die hier vorgeschlagenen Vorrichtungen und Verfahren auch als Schnelltest Einsatz finden, um gezielt Resistenzen in bereits isolierten und identifizierten Erregern zu entdecken. So können auch Veränderungen, etwa neue Resistenzen in erkannten Erregern festgestellt werden.

Vorrichtung 20 umfasst ein Heizelement 32, das ausgebildet ist, um den Probenträger 16 und mithin die Probe 14 und die resistenten Keime 12 auf eine konstante oder variierende Temperatur zu erwärmen. Das Heizelement 32 ist ausgebildet, um den Probenträger 16 auf eine Temperatur in einem Bereich zwischen 25° Celsius und 45° Celsius zu heizen. Die Erwärmung kann beispielsweise genutzt werden, um optimale, d.h. dem Körper des Patienten, bzw. der Umgebung, aus welcher die Probe entnommen wurde, nachempfundene Temperaturen, wie etwa 37° Celsius, nachzubilden, um eine optimale Vermehrung der Keime 12 zu ermöglichen. Alternativ kann das Heizelement ausgebildet sein, den Probenträger auf eine keimspezifische Temperatur zu erwärmen, wenn die Probe 14 auf einen bestimmten Erreger untersucht werden soll.

Die Erwärmung kann zu einer weiteren Verkürzung der Testzeit und mithin zu einem dritten Zeitvorteil führen. Alternative Ausführungsbeispiele zeigen Heizelemente, die ausgebildet sind, um den Probenträger auf eine Temperatur in einem Bereich zwischen 30° Celsius und 42° Celsius oder zwischen 35° Celsius und 40° Celsius zu heizen. Dies ermöglicht die Inkubation bzw. das Aufheizen auf die Temperatur für die Nachweisreaktion direkt in der Vorrichtung 20.

Alternativ oder zusätzlich kann das Heizelement 32 ausgebildet sein, um den Probenträger 16 auf eine Temperatur zu heizen, die größer als 70° Celsius, größer 100° Celsius oder größer als 200° Celsius ist, um die Probe 14 zu zerstören und/oder die Probe oder den Probenträger 16 zu desinfizieren.

Vorrichtung 20 weist ferner eine Bewegungseinrichtung 49 zum Schütteln des Probenträgers 16 auf. Bei der Einrichtung 49 kann es sich beispielsweise um einen Piezo- oder Elektromotor handeln. Ein Schütteln des Probenträgers 16 kann eine Vermischung des Nährmittels 17, des Mittels 18 und/oder des Färbemittels 36 sowie der Probe 14 ermöglichen, so dass ganz oder teilweise ein Stoffgemisch entsteht, welches gemeinsam von der Analyseeinrichtung 22 analysiert wird. Ein Stoffgemisch ermöglicht eine verbesserte Einwirkung des Mittels 18 auf die Keime der Probe 14, eine verbesserte Ernährung der resistenten Keime 12 mittels des Nährmediums 17 sowie eine homogenere Einwirkung des Färbemittels 36.

Alternativ kann Vorrichtung 20 oder der Probenträger 16 auch manuell geschüttelt werden, bevor der Probenträger 16 mit der Vorrichtung 20 kontaktiert, d.h. in diese eingeführt wird oder wenn der Probenträger 16 mit der Vorrichtung 20 kontaktiert ist und Vorrichtung 20 ein Handgerät ist.

Vorrichtung 20 weist einen optionalen Temperaturfühler 46 auf, der ausgebildet ist, um die Temperatur in dem Probenträger 16 zu erfassen. Vorrichtung 20 weist ferner eine optionale Berechnungseinrichtung 48 auf, die ausgebildet ist, die erfasste Temperatur von dem Temperaturfühler 46 zu erfassen und das Heizelement 32 anzusteuern, so dass ein Temperaturregelkreis implementierbar ist. Vorteilhaft an einem Temperaturregelkreis ist, dass eine Temperatur in dem Probenträger vorbestimmt variiert und nachgeregelt werden kann, beispielsweise wenn über verschiedene durchgeführte Tests verschiedene Mengen an Probenmaterial, d.h. verschieden große Proben 14 in unterschiedlichen Probenträger 16 in die Vorrichtung 20 eingebracht werden.

Alternative Ausführungsbeispiele zeigen Vorrichtungen, die einen oder mehrere zusätzliche Behälter für Flüssigkeiten und/oder Gase sowie Zuleitungen aufweisen und ausgebildet sind, die Flüssigkeiten und/oder Gase mit der Probe zu kontaktieren. So kann bspw. eine Begasung der Probe und mithin der Keime mit dem jeweiligen Gas, wie etwa Sauerstoff, Stickstoff und/oder Kohlenstoffdioxid erfolgen, um die Inkubation der Keime weiterer zu beschleunigen. Ferner kann mit der Flüssigkeit, bspw. Wasser, eine Luftfeuchtigkeit in einem Bereich zwischen bspw. 20 % - 100 %, 30 % - 90 % oder 40 % - 80 % um die Probe herum eingestellt werden, um die Inkubation der Keime zu beschleunigen.

Alternative Ausführungsbeispiele weisen ferner Sensoren zur Feuchtigkeitsmessung, bspw. ein Hygrometer, oder Gaskonzentrationsmessung, bspw. ein Gasspektrometer, auf, um die Luftfeuchte und/oder die Gaskonzentration im Umfeld der Probe zu erfassen. Die Sensoren können mit einer Prozessoreinrichtung oder der Berechnungseinrichtung 48 verbunden sein, um einen Regelkreis zur Steuerung der Luftfeuchtigkeit oder der Gaskonzentration zu ermöglichen.

Obwohl in obigen Ausführungen die Beleuchtungseinrichtung 42 als Teil der Vorrichtung 20 beschrieben wurde, kann die Beleuchtungseinrichtung 42 auch ein Teil der Analyseeinrichtung 22 sein, bspw. wenn die Analyseeinrichtung ein Konfokalmikroskop zur Erfassung der Lichtemission 24 aufweist.

Alternativ oder zusätzlich ist ebenfalls vorstellbar, dass Vorrichtung 20 ausgebildet ist, mit einem weiteren Probenträger kontaktiert zu werden. In oder an dem weiteren Probenträger kann eine Referenzprobe angeordnet sein. Die Referenzprobe kann beispielsweise so ausgebildet sein, dass die keine resistenten Keime 12 aufweist. Die Referenzprobe kann mit dem Nährmedium 17, dem Mittel 18 und/oder dem Färbemittel 36 kontaktiert werden. Die Analyseeinrichtung 22 kann ausgebildet sein, die Probe 14 und die die Referenzprobe zu analysieren. Das Färbemittel kann zu einem Zeitpunkt mit der Probe 14 und der Referenzprobe kontaktiert werden, zu dem das Mittel 18 keine oder nicht alle lebenden Keime in der Probe und/oder der Referenzprobe abgetötet hat, d. h. auch nicht-resistente Keime oder deren Stoffwechselprodukte werden markiert und tragen zur Lichtemission 24 bei. Basierend auf dem Mittel 18 kann die Lichtemission in der Referenzprobe stärker absinken als in der Probe 14, wenn die nicht-resistenten Keime absterben und nur (über-)lebende Keime zur weiteren Lichtemission beitragen. Die Lichtemission der Referenzprobe kann somit einen veränderlichen, dynamischen Schwellwert darstellen. Unterscheiden sich mit zunehmender Zeitdauer, in welcher das Mittel 18 mit der Probe 14 und der Referenzprobe kontaktiert ist, die Lichtemission 24 und die Lichtemission der Referenzprobe zunehmend, kann dies ein Indiz auf eine Anwesenheit resistenter Keime 12 in der Probe 14 sein. Dies kann zu einem weiteren Zeitvorteil führen, wenn auf ein Abwarten eines Eintretens einer Wirkung des Mittels 18 ganz oder teilweise verzichtet werden kann, bis mit einer Analyse der Probe 14 begonnen wird.

Ein Nachweis der resistenten Keime kann durch einen Vergleich der Lichtemission 24 mit einer Lichtemission der Referenzprobe erfolgen, wenn bspw. die Probe 14 gleich oder ähnlich wie die Referenzprobe inkubiert wird und die Lichtemission 24 größer ist, als die Lichtemission der Referenzprobe. In anderen Worten kann eine Küvette, die die Referenzprobe enthält von ihrer Beschaffenheit so gestaltet sein, dass sie sich im Wesentlichen von der zu analysierenden Probe nicht unterscheidet, außer dass keine multiresistenten Erreger enthalten sind (MRE-negative Probe), als weiterer Probenträger mit Vorrichtung 20 kontaktiert werden, so dass die Vorrichtung 20 ausgebildet sein kann, mit zwei oder mehreren Probenträger gleichzeitig oder nacheinander kontaktiert zu werden. Dies ermöglicht einen Vergleich der Lichtemission 24 mit einem dynamischen Schwellwert, der individuell für jede zu analysierende Probe 14 sein kann.

In anderen Worten haben die hier beschriebenen Verfahren und Vorrichtungen zum Ziel, multiresistente Erreger vergleichsweise kostengünstig und innerhalb kurzer Zeit nachzuweisen, so dass das Übertragungsrisiko der MRE auf andere Patienten deutlich verringert werden kann. Die entsprechende Vorrichtung kann so aufgebaut sein, dass sie sich zum Schnelltest, beispielsweise als ein Handgerät, bei der Eingangsuntersuchung bzw. Patientenaufnahme in einer medizinischen Einrichtung eignet.

Der Nachweis der MRE kann durch die Messung der enzymatischen Aktivität von den überlebenden Erregern gebildeter Proteine (z.B. Virulenzfaktoren wie DNase, Katalase, Hyaluronidase o.a. aber auch Enzyme des biochemischen Stoffwechsels wie z.B. β-Galaktosidase, α-Amylase o.a.) und/oder durch die Bestimmung der Integrität ihrer Membranen unter Verwendung von Fluoreszenzindikatoren oder Farbindikatoren erfolgen.

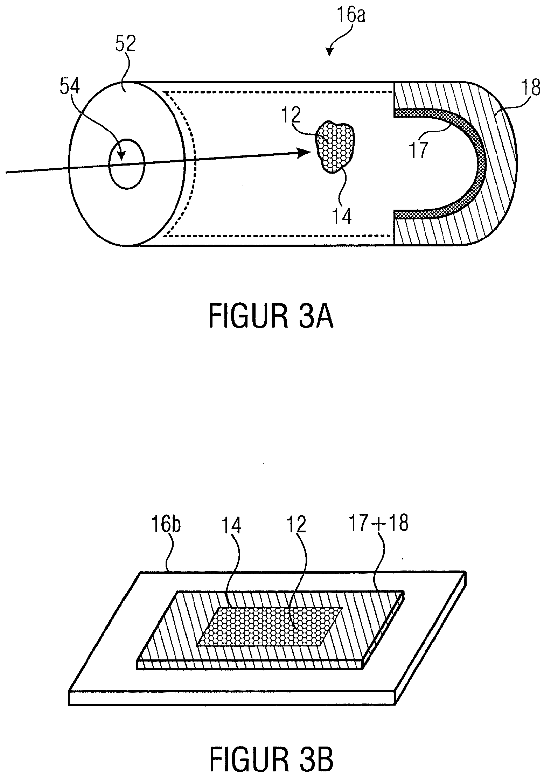

Fig. 3a zeigt eine beispielhafte Ausführungsform eines Probenträgers 16a, der als ein nahezu geschlossenes Volumen ausgeführt ist. der Probenträger 16a weist einen Verschluss 52 auf, der von dem restlichen Probenträger 16a entfernt werden kann. So kann beispielsweise nach einem erfolgten Test der Probenträger 16a gereinigt werden und/oder desinfiziert werden. Auch kann eine neue Probe 14, neues Mittel 18 und/oder Nährmittel 17 in den Probenträger 16a eingebracht, dieser mit dem Verschluss 52 verschlossen werden und ein neuer Test durchgeführt werden. Der Verschluss 52 weist eine Öffnung 54 auf, die ausgebildet ist, um eine Einführung der Probe 14 durch den Verschluss 52 hindurch zu ermöglichen, so dass beispielsweise ein mit der Probe 14 beladener Abstrichtupfer in den Probenträger 16a eingetaucht und die Probe 14 an die Mittel 18 und/oder dem Nährmittel 17 abgestrichen oder von dem Färbemittel abgespült werden kann. Alternativ oder zusätzlich kann die Öffnung 54 auch verschlossen sein und einen für Zuflussleitungen oder Kanülen, etwa für Nährmittel 17 und/oder Färbemittel permeable Komponente aufweisen, etwa eine Gummischicht. Alternativ kann das Färbemittel, das Nährmittel 17 und/oder das Mittel 18 eine miteinander vermischtes Stoffgemisch oder eine Flüssigkeit sein, so dass die Probe 14 beispielsweise durch ein Rühren eines Tupfers in dem Stoffgemisch oder der Flüssigkeit in den Probenträger 16a gerührt oder gespült wird.

Alternativ kann der Verschluss 52 auch fest an dem Probenträger 16a angeordnet sein, beispielsweise wenn der Probenträger 16a lediglich für eine einmalige Verwendung ausgelegt ist. Die Öffnung 54 kann durch einen weiteren Verschluss, wie etwa einen Pfropfen, verschlossen werden.

Fig. 3b zeigt eine schematische Darstellung eines Probenträgers 16b, der als Platte ausgeführt ist. An einer Hauptseite, d.h. einer flächenmäßig großen oder größten Oberfläche des Probenträgers 16b ist das Nährmittel 17 und das Mittel 18 zum Abtöten verschiedener Keime als Stoffgemisch angeordnet. Auf dem Stoffgemisch ist die Probe 14 mit den multiresistenten Keimen 12 angeordnet. Eine Ausführung des Probenträgers 16b als Platte kann beispielsweise zu einem gegenüber dem Probenträger 16a in Fig. 3a verringerten Bauraum führen, der auch zu einer kleineren Ausgestaltung der Vorrichtung 10 oder 20 aus den Fig. 1 oder 2 führen kann. Der Probenträger 16b kann bspw. ein Teststreifen mit einer Schicht Agar als Nährmedium 17 sein. Auf den Probenträger 16b kann beispielsweise eine Platte oder Abdeckung angeordnet sein, die ausgebildet ist, um eine Kontamination zu verhindern. Die Kontamination kann bspw. durch einen Übertritt der multiresistenten Keime 12 in die oder durch einen Übertritt von Teilen oder Mitteln, bspw. Desinfektionsmittel oder Schmiermittel, in die Probe 14 erfolgen. Prinzipiell kann der Probenträger 16 eine beliebige Form aufweisen.

Obwohl in vorangegangenen Ausführungsbeispielen das Färbemittel über eine Zuleitung mit der Probe kontaktiert wurde, ist es ebenfalls vorstellbar, dass das Färbemittel als Nachweisreagenzie mit dem Mittel zur Abtötung mehrerer Keime und/oder dem Nährmedium in oder an dem Probenträger angeordnet ist oder ein gemeinsames Mittel zur Abtötung mehrerer Keime oder Stoffgemisch mit dem Mittel zur Abtötung mehrerer Keime und/oder dem Nährmedium ist.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

### Literatur

[1] Kayser F. H., K. A. Bienz, J. Eckert, R. M. Zinkernagel; Medizinische Mikrobiologie; Georg Thieme-Verlag, Stuttgart- New York (1998) 9. Auflage
[2] Brandis H., W. Köhler, H. J. Eggers, G. Pulverer; Lehrbuch der medizinischen Mikrobiologie; Gustav Fischer Verlag, Stuttgart - Jena - New York (1994) 7. Auflage
[3] Hallmann L. und F. Burkhardt, Klinische Mikrobiologie, Georg Thieme-Verlag, Stuttgart (1974) 4. Auflage
[4] Hoffelder M., A. M. Fahr, A. Turnwald-Maschler, U. Eigner; Direct detection of methicillin-resistant Staphylococcus aureus in clinical specimens by a nucleic acidbased hyhridization assay; Clin Microbiol Infect (2006) 12(12): 1163-1167
[5] Reischl U. und T. Holzmann; Aktuelle Verfahren zum Nukleinsäure geschützten Direktnachweis von MRSA; J Lab Med (2008) 32(4): 253-265
[6] Warren D. K., R. S. Liao, L. R. Merz, W. M. Jr. Dunne; Detection of methicillin-resistant Staphylococcus aureus from nasal swab specimens by a real time PCR assay; J Clin Microbiol (2004) 42(12): 5578-5581
[7] www.microphage.com
[8] www.pcds-gmbh.de
[9] Monis P.T., S. Giglio, C.P. Saint; Comparison of SYT09 and SYBR Green I for realtime polymerase chain reaction and investigation of the effect of dye concentration on amplification of DNA melting curve analysis; Anal Biochem (2005) 340: 24-34
[10] Wlodkowic D., J. Skommer, Z. Darzynkiewicz; SYTO probes in the cytometry if tumor cell death; Cytometry A (2008) 73: 496-507
[11] Wojcik K. and J. W. Dobrucki; Interaction of DNA intercalator DRAQ5, and a minor groove hinder SYTOJ7, with chromatin in live cells - influence on chromatin organization and histone-DNA interaction; Cytometry A (2008) 73: 555-562
[12] Decker E. M.; The ability of direct fluorescence-based, two-color assays to detect different physiological states of oral streptococci; Lett Appl Microbiol (2001) 33: 188-192
[13] Leeder J. S., H. M. Dosch, P. A. Harper, P. Lam, S.P. Spielberg; Fluorescence-based viability assay for studies of reactive drug intermediates; Anal Biochem (1989) 177: 364-372
[14] Liminga G., B. Jonsson, Nygren, R. Larsson; the mechanism underlying calceininduced cytotoxicity; Eur J Phannacol (1999) 383: 321-329
[15] Parthuisot N., P. Catala, K. Lemarchand, J. Baudart, P. Lebaron; Evaluation of ChemChrome V6 for bacterial viability assessment in waters; J Appl Microbiol (2000) 89: 370-380
[16] Eisenschmidt K., T. Lanio, A. Jeltsch, A. Pingoud; A fluorimetric assay for on-line detection of DNA cleavage by restriction endonucleases; J Biotechnol (2002) 96: 185-191
[17] Ghosh S. S., P. S. Eis, K. Blumeyer, K. Fearon, D. P. Millar; Real time kinetics of restriction endonuclease cleavage monitored by fluorescence resonance energy transfer; Nucleic Acids Res (1994) Vol. 22, No. 15 3155-3159
[18] Fudala R., M. E. Mummert, Z. Gryczynski, I. Gryczynski; Fluorescence detection of hyaluronidase; J Photochem Photobiol B (2011) 104:473-477
[19] Fudala R., M. E. Mummert, Z. Gryczynski, R. Rich, J. Borejdo; Lifetime-based sensing of hyaluronidase using fluorescein labeled hyaluronic acid; J Photochem Photobiol B (2012) 106: 69-73
[20] Zhang L. S. and M. E. Mummert; Development of a fluorescent substrate to measure hyaluronidase activity; Anal Biochem (2008) 379: 80-85
[21] Peak E., I. W. Chalmers, K. F. Hoffmann; Development and validation of a quantitative, high-throughput, .fluorescence-based bioassay to detect schistosoma viability; PLoS Negl Trop Dis (2010) 4:e759
[22] Guilbault G. G. and D. N. Kramer; Fluorimetric procedure for measuring the activity of dehydrogenasis; Anal Chem (1965) 37: 1219-1221
[23] Rodriguez G. G., D. Phipps, K. Ishiguro, H. F. Ridgway; Use of a fluorescent redox probe for direct visualization of actively respiring bacteria; Appl. Environ Micobiol (1992) 1801-1808

## Patentansprüche

1. Verfahren zur Erfassung resistenter Keime (12) in einer Probe (14), durch Nachweis lebender resistenter Keime durch die Messung einer enzymatischen Aktivität der von den lebenden Keimen gebildeten Proteine DNase oder Hyaluronidase, mit folgenden Schritten:
Kontaktieren der Probe (14) mit einem Probenträger (16; 16a; 16b), der ein Mittel (18) zur Abtötung unterschiedlicher Keime aufweist, das zumindest ein Antibiotikum und/oder zumindest ein Antimykotikum umfasst, und mit einem Färbemittel, das ein Fluorophor und einen Quencher aufweist und das durch das Protein aufgespalten wird, so dass das Fluorophor freigesetzt wird;
Einbringen des Probenträgers (16) in eine Analyseeinrichtung (22);
Erfassen von von dem Fluorophor ausgehenden Lichtemissionen (24) von dem Probenträger (16; 16a; 16b) durch die Analyseeinrichtung (22); und
Ausgeben einer Anzeige durch die Analyseeinrichtung (22), dass die Probe (14) zumindest einen Keim (12) enthält, der gegen das Mittel (18) zur Abtötung unterschiedlicher Keime resistent ist, falls die erfasste Lichtemission (24) einen Schwellwert übersteigt; und
Ausgeben einer Anzeige durch die Analyseeinrichtung (22), dass die Probe (14) keine Keime (12) enthält, die gegen das Mittel (18) zur Abtötung unterschiedlicher Keime resistent ist, falls die Lichtemission (24) den Schwellwert nicht überschreitet.

2. Verfahren gemäß Anspruch 1, bei dem der Probenträger (16; 16a; 16b) für den Schritt des Kontaktierens der Probe mit dem Probenträger (16; 16a; 16b) ferner ein Nährmittel für unterschiedliche Keime aufweist.

3. Verfahren gemäß Anspruch 1 oder 2, das ferner folgende Schritte umfasst:
Bereitstellen eines Foto-Multiplizierers oder eines Ein-Photon-Detektors für das Erfassen der Lichtemission;
Beleuchten des Probenträgers (16; 16a; 16b) mit einer elektromagnetischen Strahlung zur Anregung des Fluorophors zur Lichtemission.

4. Verfahren gemäß einem der vorangehenden Ansprüche, bei dem die resistenten Keime keimunspezifisch nachgewiesen werden.

5. Verfahren gemäß einem der Ansprüche 1 bis 3, das ferner folgende Schritte umfasst:
Auswerten einer Art oder eines Stamms des gegen das Mittel zur Abtötung unterschiedlicher Keime resistenten Keims;
Ausgeben einer Anzeige durch die Analyseeinrichtung, die die Art oder den Stamm des resistenten Keims anzeigt.

6. Verfahren gemäß einem der vorangegangenen Ansprüche, das ferner folgende Schritte umfasst:
Schütteln des Probenträgers (16; 16a; 16b), wenn der Probenträger (16; 16a; 16b) mit der Probe kontaktiert ist.

7. Vorrichtung (10; 20) zur Erfassung resistenter Keime in einer Probe durch Nachweis lebender resistenter Keime durch die Messung einer enzymatischen Aktivität der von den lebenden Keimen gebildeten Proteine DNase oder Hyaluronidase, mit folgenden Merkmalen:
einem Probenträger (16; 16a; 16b), der ein Mittel zur Abtötung unterschiedlicher Keime aufweist, das zumindest ein Antibiotikum und/oder zumindest ein Antimykotikum aufweist;
einem Behältnis, das ein Färbemittel beinhaltet, das ein Fluorophor und einen Quencher aufweist und das durch das Protein aufgespalten wird, so dass das Fluorophor freigesetzt wird, wobei das Behältnis ausgebildet ist, das Färbemittel an den Probenträger abzugeben;
einer Analyseeinrichtung (22), die ausgebildet ist, um von dem Fluorophor ausgehende Lichtemissionen von dem Probenträger (16; 16a; 16b) zu erfassen;
wobei die Analysevorrichtung ausgebildet ist, um eine Anzeige auszugeben, dass die Probe zumindest einen Keim enthält, der gegen das Mittel zur Abtötung unterschiedlicher Keime resistent ist, falls die Lichtemission einen Schwellwert übersteigt; und
um eine Anzeige auszugeben dass die Probe keine Keime enthält, die gegen das Mittel zur Abtötung unterschiedlicher Keime resistent sind, falls die Lichtemission den Schwellwert nicht überschreitet.

8. Vorrichtung gemäß Anspruch 7, bei der die Analyseeinrichtung einen Foto-Multiplizierer oder einen Ein-Photon-Detektor aufweist, der ausgebildet ist, Lichtemissionen von dem Fluorophor an dem Probenträger (16; 16a; 16b) zu erfassen.

9. Vorrichtung gemäß einem der Ansprüche 7 oder 8, die ferner eine Beleuchtungseinrichtung umfasst, die ausgebildet ist, um den Probenträger (16; 16a; 16b) mit einer elektromagnetischen Strahlung zur Anregung des Fluorophors zur Lichtemission zu beleuchten.

10. Vorrichtung gemäß einem der Ansprüche 7-9, bei der die Analyseeinrichtung ferner ausgebildet ist, um eine Art oder einen Stamm des gegen das Mittel zur Abtötung unterschiedlicher Keime resistenten Keims zu bestimmen und eine Anzeige auszugeben, die die Art oder den Stamm des resistenten Keims anzeigt.

11. Vorrichtung gemäß einem der Ansprüche 7-10, die ferner eine Einrichtung zum Schütteln des Probenträgers (16; 16a; 16b) aufweist.

## Claims

1. A method for detecting resistant germs (12) in a sample (14), by identification of living resistant germs through measurement of enzymatic activity of the proteins DNase or hyaluronidase formed by the living germs, comprising:
contacting the sample (14) by a sample carrier (16; 16a; 16b) comprising an agent (18) for killing different germs, wherein said agent at least includes an antibiotic and/or an antimycotic, and by a colorant comprising fluorophore and a quencher, said colorant being split by the protein such that the fluorophore is released;
introducing the sample carrier (16) in analyzing means (22);
detecting light emissions (24) originating from the fluorophore from the sample carrier (16; 16a; 16b) by the analyzing means (22); and
outputting an indication by the analyzing means (22) indicating that the sample (14) contains at least one germ (12) resistant to the agent (18) for killing different germs, if the detected light emission (24) exceeds a threshold value; and
outputting an indication by the analyzing means (22) indicating that the sample (14) does not contain germs (12) resistant to the agent (18) for killing different germs, if the light emission (24) does not exceed the threshold value.

2. The method in accordance with claim 1, wherein, for the step of contacting the sample by the sample carrier (16; 16a; 16b), the sample carrier (16; 16a; 16b) additionally comprises a nutrient for different germs.

3. The method in accordance with claim 1 or 2, further comprising:
providing a photomultiplier or a single-photon detector for detecting the light emission;
illuminating the sample carrier (16; 16a; 16b) by electromagnetic radiation for exciting the fluorophore for light emission.

4. The method in accordance with any of the preceding claims, wherein the resistant germs are identified in a germ-unspecific manner.

5. The method in accordance with any of claims 1 to 4, comprising:
evaluating a kind or strain of the germ resistant to the agent for killing different germs;
outputting an indication by the analyzing means indicating the kind or strain of the resistant germ.

6. The method in accordance with any of the preceding claims, further comprising:
shaking the sample carrier (16; 16a; 16b) when the sample carrier (16; 16a; 16b) is contacted by the sample.

7. A device (10; 20) for detecting resist germs in a sample by identification of living resistant germs through measurement of enzymatic activity of the proteins DNase or hyaluronidase formed by the living germs, comprising:
a sample carrier (16; 16a; 16b) comprising an agent for killing different germs, wherein said agent at least includes an antibiotic and/or an antimycotic;
a container containing a colorant comprising fluorophore and a quencher and being split by the protein such that the fluorophore is released, wherein the container is configured to provide the colorant to the sample carrier;
an analyzing means (22) configured to detect light emissions originating from the fluorophore by the sample carrier (16; 16a; 16b);
wherein the analyzing means is configured to output an indication indicating that the sample contains at least one germ resistant to the agent for killing different germs, if the light emission exceeds a threshold value; and
to output an indication indicating that the sample does not contain germs resistant to the agent for killing different germs, if the light emission does not exceed the threshold value.

8. The device in accordance with claim 7, wherein the analyzing means comprises a photomultiplier or a single-photon detector configured to detect light emissions from the fluorophore on the sample carrier (16; 16a; 16b).

9. The device in accordance with any of claims 7 or 8, further comprising illuminating means configured to illuminate the sample carrier (16; 16a; 16b) with electromagnetic radiation for exciting the fluorophore for light emission.

10. The device in accordance with any of claims 7 to 9, wherein the analyzing means is further configured to determine a kind or strain of the germ resistant to the agent for killing different germs and to output an indication indicating the kind or strain of the resistant germ.

11. The device in accordance with any of claims 7 to 10, further comprising means for shaking the sample carrier (16; 16a; 16b).

## Revendications

1. Procédé de détection de germes résistants (12) dans un échantillon (14) grâce à la démonstration de germes résistants vivants par mesure d'une activité enzymatique des protéines DNase ou hyaluronidase formées par les germes vivants, aux étapes suivantes consistant à:
mettre l'échantillon (14) en contact avec un porte-échantillons (16; 16a; 16b) qui présente un agent (18) destiné à tuer différents germes qui comporte au moins un antibiotique et/ou au moins un antimycosique, et avec un colorant qui présente un fluorophore et un agent désactivateur et qui est décomposé par la protéine de manière que soit libéré le fluorophore;
introduire le porte-échantillons (16) dans un moyen d'analyse (22);
détecter par le moyen d'analyse (22) les émissions de lumière (24) issues du fluorophore du porte-échantillons (16; 16a; 16b); et
sortir par le moyen d'analyse (22) une indication du fait que l'échantillon (14) contient au moins un germe (12) qui est résistant à l'agent (18) destiné à tuer différents germes si l'émission de lumière détectée (24) excède une valeur de seuil; et
sortir par le moyen d'analyse (22) une indication du fait que l'échantillon (14) ne contient pas de germes (12) qui sont résistants à l'agent (18) destiné à tuer différents germes si l'émission de lumière (24) n'excède pas la valeur de seuil.

2. Procédé selon la revendication 1, dans lequel le porte-échantillons (16; 16a; 16b) présente par ailleurs, pour l'étape consistant à mettre l'échantillon en contact avec le porte-échantillons (16; 16a; 16b), une substance nutritionnelle pour différents germes.

3. Procédé selon la revendication 1 ou 2, comportant par ailleurs les étapes suivantes consistant à:
prévoir un photomultiplicateur ou un détecteur à un photon destiné à détecter l'émission de lumière;
éclairer le porte-échantillons (16; 16a; 16b) à l'aide d'un rayonnement électromagnétique pour exciter le fluorophore en vue de l'émission de lumière.

4. Procédé selon l'une des revendications précédentes, dans lequel les germes résistants sont démontrés de manière non spécifique aux germes.

5. Procédé selon l'une des revendications 1 à 3, comportant par ailleurs les étapes suivantes consistant à:
évaluer une espèce ou une souche du germe résistant à l'agent destiné à tuer différents germes;
sortir, par l'analyseur, un affichage qui indique l'espèce ou la souche du germe résistant.

6. Procédé selon l'une des revendications précédentes, comportant par ailleurs les étapes suivantes consistant à:
secouer le porte-échantillons (16; 16a; 16b) lorsque le porte-échantillons (16; 16a; 16b) est mis en contact avec l'échantillon.

7. Dispositif (10; 20) de détection de germes résistants dans un échantillon grâce à la démonstration de germes résistants vivants par la mesure d'une activité enzymatique des protéines DNase ou hyaluronidase formées par les germes vivants, aux caractéristiques suivantes:
un porte-échantillons (16; 16a; 16b) qui présente un agent destiné à tuer différents germes qui présente au moins un antibiotique et/ou au moins un antimycosique;
un réceptacle qui contient un colorant qui présente un fluorophore et un agent désactivateur et qui est décomposé par la protéine de manière que soit libéré le fluorophore, où le réceptacle est conçu pour délivrer le colorant vers le porte-échantillons;
un moyen d'analyse (22) qui est conçu pour détecter les émissions de lumière du porte-échantillons (16; 16a; 16b) sorties du fluorophore;
dans lequel le dispositif d'analyse est conçu pour sortir une indication du fait que l'échantillon contient au moins un germe qui est résistant à l'agent destiné à tuer différents germes si l'émission de lumière excède une valeur de seuil; et
pour sortir une indication du fait que l'échantillon ne contient pas de germes résistants à l'agent destiné à tuer différents germes si l'émission de lumière n'excède pas la valeur de seuil.

8. Dispositif selon la revendication 7, dans lequel le moyen d'analyse présente un photomultiplicateur ou un détecteur à un photon qui est conçu pour détecter les émissions de lumière du fluorophore vers le porte-échantillons (16; 16a; 16b).

9. Dispositif selon l'une des revendications 7 ou 8, qui comporte par ailleurs un moyen d'éclairage qui est conçu pour éclairer le porte-échantillons (16; 16a; 16b) par un rayonnement électromagnétique pour exciter le fluorophore en vue de l'émission de lumière.

10. Dispositif selon l'une des revendications 7 à 9, dans lequel le moyen d'analyse est par ailleurs conçu pour déterminer une espèce ou une souche du germe résistant à l'agent destiné à tuer différents germes et pour sortir un affichage qui indique l'espèce ou la souche du germe résistant.

11. Dispositif selon l'une des revendications 7 à 10, présentant par ailleurs un moyen destiné à secouer le porte-échantillons (16; 16a; 16b).
